# EUROPÄISCHE PATENTSCHRIFT

(11) **EP 1 486 218 B1**
(45) Veröffentlichungstag und Bekanntmachung des Hinweises auf die Patenterteilung: **06.12.2017**
(21) Anmeldenummer: 04018834.4
(22) Anmeldetag: 07.09.1999
(51) Int. Cl.: A61M 5/145, A61B 5/00, A61M 5/172, A61B 5/145, G01N 33/487, G01N 33/49, G06F 19/00, H05K 7/10, A61M 5/142, A61M 5/168

(54) **System zum Fernbedienen und Überwachen einer Verabreichungsvorrichtung**
Remote control and monitoring device for a drug delivery device
Système de commande à distance et de surveillance d'un dispositif d'administration de médicaments

(30) Priorität: 08.09.1998 DE 19840965
(43) Veröffentlichungstag der Anmeldung: 15.12.2004
(62) Teilanmeldung aus: 99939890.2
(73) Patentinhaber: F. Hoffmann-La Roche AG, 4070 Basel (CH); Roche Diabetes Care GmbH, 68305 Mannheim (DE)
(72) Erfinder: Haueter, Ulrich, 3506 Grosshöchstetten (CH); Imhof, Erich, 3427 Utzenstorf (CH)
(74) Vertreter: Rentsch Partner AG

(56) Entgegenhaltungen:
- EP-A- 0 048 423
- EP-A- 0 183 351
- EP-A- 0 317 705
- WO-A1-97/21456
- US-A- 5 630 710
- US-A- 5 713 856
- US-A- 5 764 159

## Beschreibung

Die Erfindung betrifft ein System zum Fernbedienen und Überwachen einer Verabreichungsvorrichtung zur Selbstverabreichung eines Produktfluids. Die Erfindung wird durch die Merkmale von Anspruch 1 definiert.
Der Überwachung des Gesundheitszustands von Menschen kommt zunehmende Bedeutung zu. Insbesondere gilt dies für die Durchführung von Therapien, die eine dosierte Verabreichung eines oder mehrerer Wirkstoffe in Abhängigkeit von dem gesundheitlichen Zustand der betreffenden Person erfordern. Als Beispiel sei die Diabetestherapie genannt, wo die Verabreichung von Insulin in Abstimmung mit der gemessenen bzw. durch Messung ermittelten Glukosekonzentration in einer Körperflüssigkeit der Person erfolgt.

Systeme zum Bedienen und Überwachen einer Verabreichungsvorrichtung zur Selbstverabreichung eines Produktfluids sind aus der EP 0 183 351 A1, EP 0 317 705 A1, EP 0 048 423 A2, US 5 630 710 A, US 5 764 159 A, WO 97/21456 A1 und US 5 713 856 A bekannt.

Die aus EP 0 183 351 A1, EP 0 317 705 A1, US 5 630 710 A, US 5 764 159 A bekannten Systeme haben eine Fernbedienung. Die aus WO 97/21456 A1 und US 5 713 856 A Bekannten haben eine Anzeige, die die Fluidmenge über der Zeit als Förderrate aufgetragen darstellen kann. Die Überwachungsverfahren gerade im Rahmen von Therapien erfordern Spezialgerät allein für den Zweck, Messdaten für einen behandelnden Arzt oder die zu behandelnde Person verfügbar zu maschen. Die Geräte zur Überwachung sind komplex und teuer oder gestatten eine Überwachung nur in einem sehr eingeschränkten Ausmaß. In der Diabetestherapie, wo die Glukosekonzentration in einer Körperflüssigkeit, insbesondere der Blutzuckergehalt, maßgebend ist, wird mit bekannten Vorrichtungen zur Überwachung der Glukosekonzentration nur die aktuell gemessene Konzentration angezeigt. Zumindest sind die Auswerte- und Anzeigemöglichkeiten sehr eingeschränkt. Ein Benutzer, der sich das Insulin, oder in einer anderen Therapie den entsprechenden anderen Wirkstoff, selbst verabreicht, könnte die Verabreichung seinen persönlichen Bedürfnisse genauer anpassen, wenn ihm beispielsweise auch "historische" Daten zur Verfügung stünden. Er könnte seine körperlichen Aktivitäten, Essgewohnheiten und zu verabreichenden Wirkstoffdosen genauer aufeinander abstimmen.

Eine Messwertaufnahme, die einen Messwert aufnimmt und für eine Überwachung eines Gesundheitszustands einer Person verfügbar macht, als Modul für eine Schnittstelle eines herkömmlichen Computers ausgebildet wird. Bei dem genannten Messwert handelt es sich um einen Messwert, der mittels eines Sensors an der Person zur Ermittlung des Gesundheitszustands aufgenommen worden ist. Das Modul ist für den Einbau und Ah Schluss an eine definierte Schnittstelle des Computers vorbereitet. Es kann somit nicht nur vom Computerhersteller, sondern auch nachträglich von unabhängigen Herstellern bereit gestellt werden.
In einer besonders bevorzugten Verwendung handelt es sich bei dem Modul um ein Modul, das in der Überwachung der Glukosekonzentration in einer Körperflüssigkeit der Person, insbesondere in der Überwachung des Blutzuckergehalts, eingesetzt wird.
Ein Vorteil der Modulbauweise ist, dass herkömmliche Computer für die Überwachung verfügbar gemacht werden, die im allgemeinen über eine weit höhere Leistungsfähigkeit, insbesondere in Bezug auf Rechenleistung und Darstellungsmöglichkeiten, verfügen als bislang in der Überwachung eingesetzte Spezialgeräte. Ein weiterer Vorteil ist, dass insbesondere in Therapieformen, in denen eine Person sich den entsprechenden Wirkstoff selbst verabreicht, diese Person auf einen im allgemeinen bereits verfügbaren Computer zurückgreifen kann, den sie für andere Zwecke bereits benutzt und an dessen Betrieb sie gewöhnt ist. Ebenso ist die Übergabe von Gesundheitsdaten vom Modul zu anderen, weitverbreiteten Computerprogrammen möglith. Das Modul bzw. dessen Software für den Computer ist zu den üblichen Programmen, wie beispielsweise Graphik-, Text- und/oder Tabellenkalkulationsprogrammen, kompatibel. Besonders bevorzugt ist das Modul ein Steckmodul, das vorteilhafterweise in einen Standardsteckplatz eines Computers einsteckbar ist, beispielsweise in der Form der heutzutage üblichen Standard-Steckkarten für einen äußeren Port eines Computers. Es kann sich auch um ein Steckmodul handeln, das nachträglich auf einem der verfügbaren Steckplätze im Gehäuse eines PC eingebaut wird. In jedem Falle jedoch wird die im Computer vorhandene Kapazität genutzt. Es sind insbesondere die Prozessor- und Bildschirmkapazitäten, aber auch die bereits vorhandenen Möglichkeiten für den Anschluss von Perpheriegeräten, die erschlossen werden. Der Benutzer kann daher zur Überwachung des Gesundheitszustands auf die ihm bekannte Computerumgebung zurückgreifen, was der Akzeptanz der Überwachung zugute kommt. Vorzugsweise wird mit solch einem Steckmodul die entsprechende Installations- und Anwendungssoftware für die Überwachung mitgeliefert, wie dies bei nachrüstbaren Steckmodulen im allgemeinen üblich ist.

Weitere vorteilhafte Merkmale einer in ein Modul integrierten Messwertaufnahme werden durch die Unteransprüche beschrieben.

Die Anordnung findet besonders bevorzugt Verwendung bei einem Computer einer Vorrichtung zur Selbstverabreichung eines Produktfluids oder eines in Verbindung damit eingesetzten Computers. Die Vorrichtung umfasst eine Fluidführungseinrichtung, ein Fördermittel zur Förderung einer zu verabreichenden Produktfluiddosis, eine Schnittstelle für drahtlose Kommunikation, eine Steuerung für das Fördermittel und ein Kommunikations-Endgerät. Die Fluidführungseinrichtung, das Fördermittel und die genannte Schnittstelle sind Bestandteile eines Verabreichungsgeräts. Das Kommunikations-Endgerät seinerseits ist von diesem Gerät körperlich losgelöst, d.h. zumindest bei Benutzung der Vorrichtung sind diese beiden Geräte mechanisch nicht miteinander verbunden. Eine etwaige Verbindung bei der Lagerung oder dem Transport soll jedoch nicht ausgeschlossen sein. Das Kommunikations-Endgerät ist mit einer weiteren Schnittstelle für drahtlose Kommunikation und einer optischen Anzeige ausgestattet. Zwischen dem Verabreichungsgerät und dem Kommunikations-Endgerät findet eine Kommunikation drahtlos über diese beiden Schnittstellen statt. Das Verabreichungsgerät und das Kommunikations-Endgerät verbleiben bei dem Benutzer, der sich das Produktfluid in eigener Regie verabreicht.

Die Fluidführungseinrichtung umfasst ein Reservoir für das Produktfluid und die damit verbundenen und vom Fluid bei der Verabreichung durchströmten Teile des Verabreichunggeräts bis hin zu einer Fluidaustrittsstelle. Im Falle eines am Körper oder an der Kleidung zu befestigenden Verabreichungsgeräts wird die Fluidaustrittsstelle von einer Einstechnadel gebildet.

In einer ebenfalls bevorzugten Mischform sind das Fördermittel, ein Notsteuerungsteil hierfür, eine Energiequelle und die Schnittstelle für drahtlose Kommunikation implantiert, und das Reservoir wird am Körper oder an der Kleidüng befestigt, wobei die Fluidführungseinrichtung stromab vom Reservoir naturgemäß teilweise ebenfalls implantiert ist; das Fördermittel, vorzugsweise eine Mikropumpe, ist dabei vorzugsweise selbst Teil des Fluidführungssystems, was es im übrigen grundsätzlich in den anderen, bezüglich des Aufbaus des Verabreichungsgeräts alternativen Ausführungsformen ebenfalls sein kann. Vorteile der Aufteilung des Verabreichungsgeräts in körperexternes Reservoir und körperinternes Fördermittel ist, dass der externe Teil verkleinert oder das Reservoir vergrößert werden kann und dass der Ort der Ausschüttung des Produktfluids für die Therapie besonders günstig gewählt werden kann. Gleichzeitig wird das Gesundheitsrisiko, das mit einem implantierten Reservoir verbunden ist, vermieden.

Das Produktfluid ist vorzugsweise eine flüssige Wirkstofflösung, wie sie im Rahmen einer Therapie verabreicht wird, insbesondere Insulin.

Die drahtlose Kommunikation erfolgt vorzugsweise über Funk. Aber auch eine Ultraschall-oder Infrarotkommunikation kommt in Betracht.

Auf der Anzeige des Kommunikations-Engeräts wird wenigstens ein Betriebsparameter des Verabreichungsgeräts dargestellt. Eine sich das Produktfluid selbst verabreichende Person kann den Betrieb des Verabreichungsgeräts und damit die Verabreichung unmittelbar und bequem am Kommunikations-Endgerät überwachen. Der Benutzer braucht das Verabreichungsgerät nicht zum Ablesen einer Anzeige zu lösen, da er die ihn interessierenden Informationen auf der Anzeige des bereits losgelösten Kommunikations-Endgeräts unabhängig vom Ort der Anbringung des Verabreichungsgeräts ablesen kann. Das Verabreichungsgerät kann hierdurch für einen Verabreichungszyklus bis zum nächsten Befüllen des Reservoirs an jeder geeigneten Stelle an der Kleidung des Benutzers oder unmittelbar am Körper befestigt werden. Bereits hierdurch erhöht sich der Tragekomfort für den Benutzer. Das Verabreichungsgerät kann für andere Personen nicht sichtbar ständig unter der Kleidung, beispielsweise unter einem Pullover, getragen werden. Dies erhöht die Akzeptanz des Geräts. Das erleichterte Ablesen erhöht gleichzeitig die Sicherheit, u. a. da der Benutzer deshalb seine Anzeige öfter prüft. Anzeigemittel am Verabreichungsgerät können vorgesehen sein, sind aber nicht mehr erforderlich und vorzugsweise am Verabreichungsgerät nicht oder nur in einem reduzierten Umfang vorhanden.

Ein für den Benutzer interessanter Betriebsparameter des Verabreichungsgeräts ist die verabreichte Fluidmenge. Bei einer kontinuierlichen oder quasi kontinuierlichen Produktausschüttung wird die Fluidmenge vorzugsweise über der Zeit als Förderrate aufgetragen. Insbesondere werden die in diskreten Zeitintervallen geförderten Fluidmengen dargestellt, wobei die Zeitintervalle in Anpassung an die Therapie und gegebenenfalls auch individuell für einen Benutzer geeignet vorgegeben oder wählbar sind.

Die in einem bestimmten Zeitintervall verabreichte Fluidmenge wird vorzugsweise aus der Position des Fördermittels ermittelt. Durch Vergleich einer Position am Ende eines Zeitintervalls mit einer bekannten Ausgangsposition zu Beginn des Zeitintervalls wird die im jeweiligen Zeitintervall geförderte Fluidmenge errechnet, d. h. durch Differenzbildung und Skalierung auf das Fluidreservoir. Durch Speicherung von im Zeitverlauf der Verabreichung durchlaufenen Positionen oder ermittelten Fördermengen kann die verabreichte Fluidmenge über der Zeit dargestellt werden. Die Darstellung erfolgt vorzugsweise grafisch. Es kann zur Erhöhung der Verabreichungssicherheit auch die Darstellung einer Abweichung von einer vorgegebenen Sollposition vorgesehen sein, beispielsweise ebenfalls über den Zeitverlauf der Verabreichung. Falls eine unzulässig große Abweichung von einer vorgegebenen Sollposition festgestellt wird, ist die Darstellung eines Warnsignals von Vorteil, das dem Benutzer das Auftreten einer zu beachtenden Abweichung signalisiert oder sogar das Maß der Abweichung und zweckmäßigerweise auch den Zeitpunkt des Auftretens anzeigt. Auch kann die Darstellung des zeitlichen Verlaufs der Abweichung zwischen dem Fördersoll und der tatsächlich geförderten Fluidmenge zweckmäßig sein. Aus der Position des Fördermittels kann vorteilhafterweise auch die noch verfügbare Restmenge des Produktfluids ermittelt werden, da jeder Position eine bestimmte Restmenge unmittelbar zugeordnet ist, wenn sich das Fluidreservoir in Form und Größe nicht ändert, indem beispielsweise stets gleiche Ampullen eingesetzt werden, oder Änderungen automatisch erkannt oder am Kommunikations-Endgerät manuell eingegeben werden.

Die Position des Fördermittels kann eine Sollposition sein, die von einer Steuerung vorgegeben wird, die das Fördermittel positionsgenau steuert. Nach einer ebenfalls bevorzugten Ausführungsform wird die von einem Sensor aufgenommene Istposition des Fördermittels zur Ermittlung der Fördermenge und der weiteren daraus ableitbaren Betriebsparameter verwendet.

In einer bevozugten Ausführungsform wird das Fluidführungseinrichtung in Bezug auf eine Okklusion überwacht. Das Auftreten und vorzugsweise das Ausmaß einer Okklusion werden am Kommunikations-Endgerät angezeigt. Eine Okklusion kann zum totalen Ausfall der Förderung oder zu einer Förderung mit verringerter Förderrate führen. Nach Feststellung eines Totalausfalls wird das Fördermittel automatisch stillgesetzt, und der Totalausfall wird am Kommunikations-Endgerät in Form eines Alarmsignals angezeigt. Wird trotz Okklusion weitergefördert, ist die tatsächliche Förderrate also nur geringer als eine vorgegebene Förderrate und wird festgestellt, dass diese Betriebsstörung durch eine Okklusion im Fluidführungssystem hervorgerufen wird, so wird dies vorzugsweise auf der optischen Anzeige des Kommunikations-Endgeräts angezeigt, insbesondere durch ein grafisches Warnsymbol und gleichzeitig geeigneter Darstellung der aufgetretenen Abweichung. Wie im Falle einer Abweichung aufgrund einer Fehlförderung, für die das Fördermittel ursächlich ist, kann der Benutzer bei der nächsten sich bietenden Gelegenheit ganz gezielt die Fehlförderung kompensieren. Vorzugsweise wird die Okklusion im Falle einer totalen Okklusion als Ereignis in einem Speicher des Kommunikations-Endgeräts zusammen mit dem Zeitpunkt des Feststellens gespeichert. Im Falle einer Minderförderung, deren Ausmaß aufgrund der Position des Fördermittels ermittelt werden kann, wird die Minderförderung über ihrem zeitlichen Verlauf gespeichert. Es kann, wie vorstehend bereits ausgeführt, die Darstellung der Fördermengen oder der Förderrate absolut oder die Darstellung der Abweichung von der Sollförderung vorgesehen sein.

In bevorzugter Weiterentwicklung wird eine im Fluidführungssystem aufgetretene Leckage festgestellt, zum Kommunikations-Endgerät gesendet und dort in Form eines Alarmsignals angezeigt. Vorzugsweise wird die Leckage als Ereignis in einem Speicher des Kommunikations-Endgeräts zusammen mit dem Zeitpunkt des Feststellens gespeichert und steht so für eine spätere Auswertung des Verabreichungsverlaufs zur Verfügung.

Das Auftreten einer Okklusion wird vorzugsweise mittels eines Sensors detektiert, der eine Reaktionskraft misst, die von dem Fördermittel auf eine Lagerung des Fördermittels ausgeübt wird. Die gemessene Reaktionskraft wird ständig mit einem geeignet vorgegebenen Schwellwert verglichen, dessen Überschreitung auf eine Okklusion schließen lässt. Der gleiche Sensor kann auch zur Detektion eines Lecks verwendet werden. Die gemessene Reaktionskraft wird dann zusätzlich mit einem geeignet vorgegebenen Schwellwert verglichen, dessen Unterschreitung auf eine Leckage schließen lässt.

In einem Strömungsquerschnitt des Fluidführungssystems ist vorzugsweise ein Mittel zur Erzeugung eines definierten Druckabfalls angeordnet. Der Druckabfall ist so groß bemessen, dass die Kraft, die das Fördermittel zur Überwindung dieses Strömungswiderstands aufbringen muss, deutlich größer ist als die bei ordnungsgemäßer Funktion des Verabreichungsgeräts auftretenden sonstigen, die Reaktionskraft bestimmenden Kräfte. Hierdurch wird eine Okklusion und insbesondere eine Leckage besonders sicher festgestellt.

Den höchsten Tragekomfort bietet ein Kommunikations-Endgerät, das Fernanzeige und Fernbedienung zugleich ist. Durch die Fernbedienung kann der Benutzer sein Verabreichungsgerät nicht nur diskret überwachen, sondern auch bedienen, sollte dies beispielsweise in Restaurants oder zu sonstigen gesellschaftlichen Gelegenheiten erforderlich sein. Eingabemittel des Kommunikations-Endgeräts können beispielsweise Tasten, ein Touchscreen oder eine Spracheingabe mit integriertem Mikrofon sein; letztere kann auch zusätzlich zu einem handbetätigten Eingabemittel vorgesehen sein, um eine besonders bequeme, in allen Situationen unauffällige Eingabe zu ermöglichen.

Die zur Ermittlung des wenigstens einen Betriebsparameters verwendete physikalische Größe, vorzugsweise die Position des Fördermittels und/oder die von dem Fördermittel ausgeübte Reaktionskraft, wird einem Prozessor der Vorrichtung aufgegeben, der daraus den Betriebsparameter bildet. Ist der Prozessor Bestandteil des Verabreichungsgeräts, so wird der von ihm gebildete Betriebsparameter der Schnittstelle des Verabreichungsgeräts aufgegeben und von dort zur Schnittstelle des Kommunikations-Endgeräts gesendet. Bevorzugterweise ist solch ein Prozessor Bestandteil des Kommunikations-Endgeräts, und es wird die physikalische Größe gesendet.

Eine vom Benutzer beeinflussbare Steuerung für das Fördermittel kann Bestandteil des Verabreichungsgeräts sein. Der Benutzer kann eine Einstellung dieser Steuerung mittels eines Eingabemittels des Kommunikations-Endgeräts ändern, beispielsweise zur Verabreichung eines Sonderbolus.

Nach einer bevorzugten Ausführungsform ist eine beeinflussbare Steuerung, insbesondere eine programmierbare Steuerung, oder der gesamte variierbare Teil der Steuerung des Fördermittels jedoch Bestandteil des Kommunikations-Endgeräts. Lediglich ein unumgänglich unmittelbar bei dem Fördermittel anzuordnender Teil der Steuerung, gegebenenfalls lediglich ein drahtlos gesteuertes Leistungsteil des Fördermittels, beispielsweise in Form nur eines getakteten Unterbrechers der Energiezufuhr zum Fördermittel, ist im Verabreichungsgerät untergebracht.

Bevorzugterweise umfasst das Verabreichungsgerät bei Anordnung des variierbaren Steuerungsteils im Kommunikation-Endgerät eine Notsteuerung, die bei einem Ausfall der drahtlosen Kommunikation ein festes Verabreichungsprogramm automatisch und autark bis zu einem Abschalten weiterfährt. Die Notsteuerung kann zusätzlich zu dem drahtlos angesteuerten Minimalsteuerungsteil der Hauptsteuerung vorgesehen sein. Sie kann jedoch auch durch das bei ordnungsgemäßer Funktion drahtlos gesteuerte Steuerungsteil gebildet werden, das auf Notbetrieb umschaltet, wenn innerhalb eines vorgegebenen Zeitintervalls keine Steuerungssignale ankommen. Die Notsteuerung kann nach einem für alle Verabreichungs-geräte einer Baureihe gleichen Standardprogramm arbeiten oder auch die Fähigkeit der individuellen Einstellbarkeit, vorzugsweise durch Programmierung, aufweisen, die nach Einstellung des Benutzers ebenfalls drahtlos über die Schnittstelle des Verabreichungsgeräts mittels des Kommunikations-Endgeräts oder eines anderen geeigneten Geräts vorgenommen wird, üblicherweise von einem Arzt. Eine weitergehende Veränderbarkeit weist sie jedoch nicht auf, sie fährt vielmehr das einmal eingestellte Standard-oder Individual-Programm ab.

Das Verabreichungsgerät kann sehr einfach wasserdicht gebaut werden, da ein Gehäuse wenig Durchbrechungen aufweist, wie beispielsweise für Eingabemittel. Im Extremfall müssen nur der Bereich der Schnittstelle und der Austritt für die Fluidführungseinrichtung abgedichtet werden.

Nach einer besonders bevorzugten Ausführungsform ist eine Auswerteeinrichtung für eine Überwachung des Gesundheitszustands einer Person, insbesondere des Benutzers der Vorrichtung, mit dem Kommunikations-Endgerät verbindbar oder fest integrierter Bestandteil des Kommunikation-Endgeräts. Die Auswerteeinrichtung ist vorzugsweise Bestandteil des Moduls.

Mittels der Auswerteeinrichtung wird ein aufgenommener Messwert ausgewertet, indem daraus ein entsprechender Körperwert ermittelt wird. Ein Ergebnis der Auswertung wird an einer weiteren oder vorzugsweise der bereits genannten Anzeige des Kommunikations-Endgeräts zur Anzeige gebracht. Bei Verwendung der Vorrichtung in der Insulinbehandlung handelt es sich vorzugsweise um eine Auswerteeinrichtung zum Ermitteln eines Blutzuckergehalts; vorzugsweise kann damit zusätzlich der Hormonspiegel und/oder eine relevante Körpertemperatur ermittelt werden. Grundsätzlich ist solch eine Auswerteeinrichtung jedoch nicht auf eine Auswertung von Blutzuckermesswerten bzw. der vorgenannten Messwerte und Messwertgruppen beschränkt. Im Rahmen anderer Therapien, bei denen nicht Insulin, sondern andere Wirkstoffe verabreicht werden, werden dem entsprechenden Verwendungsfall angepasst andere Messwerte aufgenommen und ausgewertet.

Der Messwert kann von einem Sensor drahtlos als Messignal gesendet werden. Die Auswerteeinrichtung umfasst eine Messwertaufnahme zum Empfang dieses Signals. Die Messwertaufnahme wird in dieser Ausführungsform durch die Schnittstelle des Kommunikations-Endgeräts gebildet oder als zusätzliches Empfangsteil ausgebildet. In einer bevorzugten anderen Ausführungsform wird ein Sensor, der mit der Probe in Berührung steht oder stand, mit seinem Kontaktbereich in die Messwertaufnahme eingeführt, die das Messignal des Sensors somit durch unmittelbaren Kontakt erhält. Die Messwertaufnahme ist in beiden Fällen das Bindeglied zwischen dem Sensor und dem Kommunikations-Endgerät oder einer anderen Plattform für die Auswerteeinrichtung.

In der Ausführung mit dem körperlich einzuführenden Sensor können der Sensor und die Auswerteeinrichtung aus der Insulintherapie bekannten Sensoren und Auswerteeinrichtungen entsprechen. Die Auswerteeinrichtung ist im Unterschied dazu jedoch kompatibel zum Kommunikations-Endgerät oder einem üblichen, jedoch der Anwendung angepassten Computer.

Die Messwertaufnahme ist integrierter Bestandteil des Moduls, insbesondere einer Standard-Steckkarte für Computer, beispielsweise einer PCMCIA Karte, und deshalb mit üblichen Computern wie PCs, Notebooks und Computern im Taschenformat einfach durch Einstecken in einen dafür vorgesehenen Steckplatz des Computers verbindbar. Die Steckkarte kann als reine Messwertaufnahme, d. h. als reines Durchleitungsglied für die Messwerte des Sensors, ausgebildet sein. Die nachfolgende Aufbereitung der Messwerte bis zum Erhalt der Körperwerte kann durch Komponenten vorgenommen werden, die der Computer von Hause aus aufweist. In dieser Ausführung ist die Steckkarte nur ein Sensorport. In einer anderen bevorzugten Ausführung sind auch Mittel zur Aufbereitung der Messwerte integrierter Bestandteil der Steckkarte. Auf der Steckkarte kann ein Permanentspeicher zum Zwischenspeichern der Messwerte oder der bereits aufbereiteten Messwerte vorhanden sein. Die Steckkarte kann mit einer vorinstallierten oder installierbaren speziellen Anwendersoftware geliefert werden.

Die Steckkarte kann ein integriertes Probennahmeset mit mehreren Sensorelementen und Mitteln zum Entnehmen von Proben beinhalten. Alternativ kann solch ein Probennahmeset aber auch Bestandteil des Kommunikations-Endgeräts sein, insbesondere dann, wenn die Auswerteeinrichtung integrierter Bestandteil des Kommunikations-Endgeräts ist.

In einer bevorzugten Ausführungsform ist das Kommunikations-Endgerät ein Standardcomputer im Taschenformat, beispielsweise ein Hand-Held oder vorzugsweise ein Palm-Sized Computer. Die Schnittstelle des Kommunikations-Endgeräts zur drahtlosen Kommunikation mit dem Verabreichungsgerät wird durch eine Steckkarte, beispielsweise eine PCMCIA Karte, für einen Steckplatz des Computers gebildet. Die gleiche Steckkarte beinhaltet vorzugsweise die Messwertaufnahme, die mit Zusatzkomponenten auf der Steckkarte bis zu einer Auswerteeinrichtung ausgebaut sein kann, und bildet so gleichzeitig Kommunikationsschnittstelle und den Sensorport.

Die Integration einer Auswerteeinrichtung oder auch nur einer Messwertaufnahme in einem Modul, vorzugsweise in einem Steckmodul, insbesondere einer Steckkarte, ist mit Vorteil auch in Verbindung mit Verabreichungsgeräten verwendbar, die ein losgelöstes Kommunikation-Endgerät nicht aufweisen. Die Modullösung kann in jeder Therapieüberwachung mit Vorteil eingesetzt werden, um die im Vergleich zu bekannten Einrichtungen größeren Rechen- und Speicherkapazitäten und auch umfangreicheren Anzeigemöglichkeiten eines Computers nutzen zu können. Als Steckmodul kann sie insbesondere ein eigenständig handelbares Produkt sein.

Vorzugsweise erfolgt bereits eine Konfigurierung und/oder eine Diagnose des Verabreichungsgeräts herstellerseitig drahtlos mittels Computer. Insbesondere erleichtert die drahtlose Kommunikation die Durchführung von Qualitätskontrollen. Da hierfür keine Drahtverbindungen hergestellt werden müssen, können Konfigurierung und Diagnose ohne Produktionsunterbrechung sozusagen bei laufendem Band durchgeführt werden, falls eine Schnittstelle für die drahtlose Kommunikation an geeigneter Stelle neben dem Band angeordnet wird.

Ein bevorzugtes Ausführungsbeispiel der Erfindung wird nachfolgend anhand von Figuren erläutert. Es zeigen:
- Figur 1: eine Vorrichtung zur Selbstverabreichung eines Produktfluids mit einem Kommunikations-Endgerät für drahtlose Kommunikation,
- Figur 2: eine Ansicht des Kommunikations-Endgeräts,
- Figur 3: ein Blockdiagramm des Kommunikations-Endgeräts,
- Figur 4: eine Touchsreen Anzeige und
- Figur 5: eine drahtlose Kommunikation zwischen dem Verabreichungsgerät und zwei alternativen Kommunikations-Endgeräten.

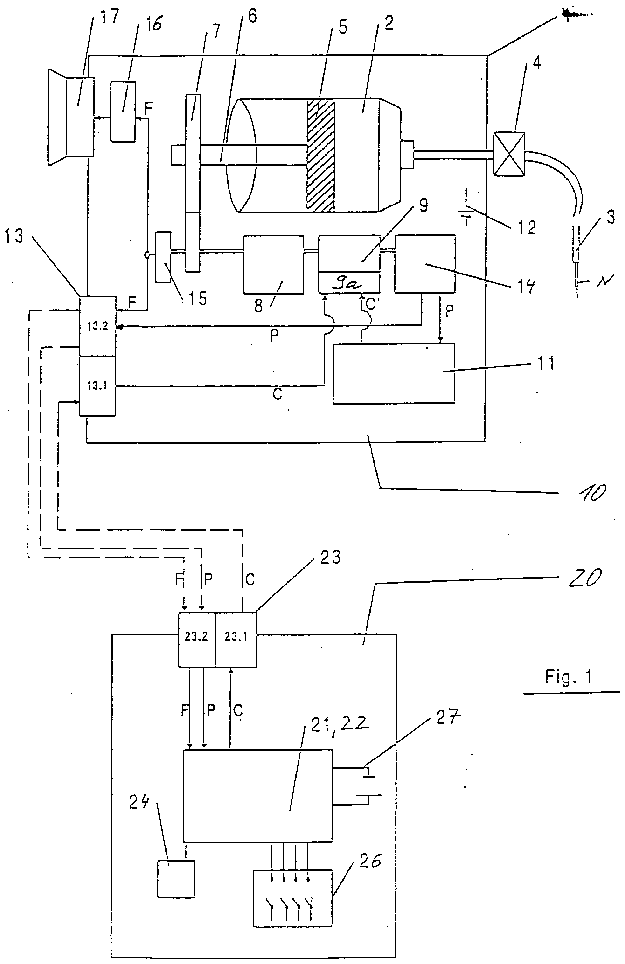

Figur 1 zeigt ein Verabreichungsgerät 10 in Form einer Infusionspumpe für Insulin, das drahtlos mit einem Kommunikations-Endgerät 20 kommuniziert.

Das Verabreichungsgerät 10 weist ein Gehäuse 1 auf, das mit geeigneten Haltemitteln versehen ist, so dass es von einem Benutzer an der Kleidung oder unmittelbar am Körper zum ständigen Tragen befestigt werden kann.

Das Insulin ist in einem Reservoir, das im Ausführungsbeispiel durch eine Ampulle 2 gebildet wird, enthalten. In der Ampulle 2 ist ein Kolben 5 verschiebbar aufgenommen. Das Verschieben des Kolbens 5 erfolgt mittels eines Spindeltriebs, dessen Abtriebsglied 6, im Ausführungsbeispiel eine Gewindestange, durch Drehantrieb eines Antriebsglieds 7, im Ausführungsbeispiel eine Gewindehülse, dem Gehäuse 1 gegenüber geradverschoben wird. Den Drehantrieb der Gewindehülse 7 bewirkt ein Schrittmotor 9, der über ein Getriebe 8 mit einem mit der Gewindehülse 7 kämmenden Stirnrad die Gewindehülse 7 drehantreibt. Ein Leistungsteil einer Steuerung für den Motor 9 ist mit 9a bezeichnet. Durch verdrehgesicherte Geradführung der Gewindestange 6 im Gehäuse 1 wird die Gewindestange 6 in axialer Richtung und gegen die Kolbenrückseite drückend verschoben. Der Kolben 5 wird unter der Einwirkung der Gewindestange 6 auf einen Ampullenauslass zu verschoben. Dadurch wird Insulin durch den Ampullenauslass verdrängt. An den Ampullenauslass angeschlossen ist eine Fluidleitung 3, an deren freien vorderen Ende eine Infusionsnadel N befestigt ist, die der Benutzer sich unter die Haut sticht und anschließend für die Selbstverabreichung des Insulins auf der Haut befestigt.

In der Fluidleitung 3 ist ebenfalls noch von dem Gehäuse 1 aufgenommen ein Ventil 4 angeordnet, das den Insulindurchfluss erst ermöglicht, wenn in der Ampulle 2 ein durch das Ventil 4 vorgegebener Mindestdruck überschritten wird.

Mit einem Positionssensor 14 wird ein Winkellagen-Istwert des Schrittmotors 9 gemessen und über Signalleitungen als Istposition P an eine Funkschnittstelle 13 und an eine im Gehäuse 1 untergebrachte Notsteuerung 11 für den Schrittmotor 9 weitergeleitet.

Die Notsteuerung 11 wird bei ordnungsgemäßer Funktion nur in einem Stand-by Modus betrieben. Die Notsteuerung 11 ist mittels Signalleitungen mit dem Positionssensor 14 und dem Leistungsteil 9a verbunden. Von dem Positionssensor 14 erhält sie die Istposition P und an das Leistungsteil 9a gibt sie ihr Stellsignal C' aus, falls die Notsteuerung 11 aktiviert ist, um den Motor 9 in eine nächste Sollposition zu fahren.
Als Energiequelle 12 für die energieverzehrenden Komponenten des Verabreichungsgeräts 10 dient eine im Gehäuse 1 untergebrachte elektrische Batterie.

Eine vom Kolben 5 auf das Gehäuse 1 ausgeübte Reaktionskraft F wird von einem Kraftsensor 15 ständig gemessen und in Form eines die gemessene Reaktionskraft F repräsentierenden Messignals ausgegeben. Die gemessene Reaktionskraft F wird über Signalleitungen der Funkschnittstelle 13 und einem Schwellwertvergleicher 16 zugeleitet, der bei Überschreitung eines vorgegebenen oberen Schwellwerts und bei Unterschreiten eines vorgegebenen unteren Schwellwerts für die gemessene Reaktionskraft F einen akustischen Alarm eines Alarmmittels 17 auslöst. Der Kraftsensor 15, der Vergleicher 16 und das Alarmmittel 17 bilden eine Einrichtung zum Auslösen eines Notalarms.

Der gesamte Antrieb des Kolbens 5, nämlich der Spindeltrieb 6, 7, das Getriebe 8, der Motor 9 mit Leistungsteil 9a, und auch der Positionssensor 14 sowie die Notsteuerung 11 sind gemeinsam auf einer im Gehäuse 1 in und gegen die Vorschubrichtung des Kolbens 5 verschiebbar geradgeführten Plattform gelagert. Die Lagerung der Plattform erfolgt in und gegen die Vorschubrichtung des Kolbens 5 frei schwimmend. Der Kraftsensor 15, beispielsweise ein DMS-Streifen in geeigneter Anordnung, ist an einer Unterseite der Plattform oder gegenüberliegend am Gehäuse 1 so angebracht, daß er bei einem Gegeneinanderdrücken der Plattform und des Gehäuses 1 ein Messignal ausgibt, das die dabei wirkende Kraft repräsentiert. Diese Kraft entspricht der vom Kolben 5 auf die Gewindestange 6 und somit auf die Plattform ausgeübten Reaktionskraft F.

Über die Funkschnittstelle 13 kommuniziert das Verabreichungsgerät 10 drahtlos mit dem Kommunikations-Endgerät 20, das im Ausführungsbeispiel als integriertes Fernanzeige- und Bediengerät ausgebildet ist. Das Kommunikations-Engerät 20 verfügt über eine Funkschnittstelle 23.

Beide Funkschnittstellen 13 und 23 weisen ie sowohl einen Empfangsteil 13.1 bzw. 23.2 als auch einen Sendeteil 13.2 bzw. 23.1 auf. Über den Sendeteil 13.1 sendet das Verabreichungsgerät 10 ständig oder periodisch die Werte der gemessenen Reaktionskraft F und der Istposition P, die vom Empfangsteil 23.2 empfangen und einem Mikroprozessor 21 über Signalleitungen zugeführt werden.

Der Prozessor ermittelt aus der Istposition P insbesondere die geförderte Basalrate, vorzugsweise in Insulineinheiten pro Stunde IU/h, Bolusabgaben in Insulineinheiten IU und den Füllzustand der Ampulle 2 und/oder die aktuell verbleibende Restmenge IUR und/oder voraussichtliche Restförderdauer. Die ermittelte Basalrate und die Bolusabgaben werden gespeichert. Die Reaktionskraft F vergleicht er mit einem vorgegebenen oberen Schwellwert für diese Kraft. Bei Überschreiten des oberen Schwellwerts wird das Auftreten einer Okklusion im Fluidführungssystem festgestellt und mit dem Zeitpunkt ihres Auftretens gespeichert. Durch Vorgabe eines unteren Schwellwerts und Vergleich damit kann auch eine Leckage im Fluidführungssystem detektiert und der Zeitpunkt ihres Auftretens gespeichert werden.

Der Prozessor 21 bildet gleichzeitig eine veränderbare Steuerung 22 für den Motor 9 im Normalbetrieb, in dem die Notsteuerung 11 im Stand-by Modus verharrt. Nur im Notfall einer Kommunikationsstörung oder eines festgestellten sonstigen Steuerungsfehlers, der zum Verlust von Steuerungssignalen führt, übernimmt die Notsteuerung 11 anstelle der Prozessorsteuerung 22 die Steuerungsfunktion. Ansonsten steuert die Prozessorsteuerung 22 den Motor durch drahtlose Übermittlung ihrer Stellsignale C.

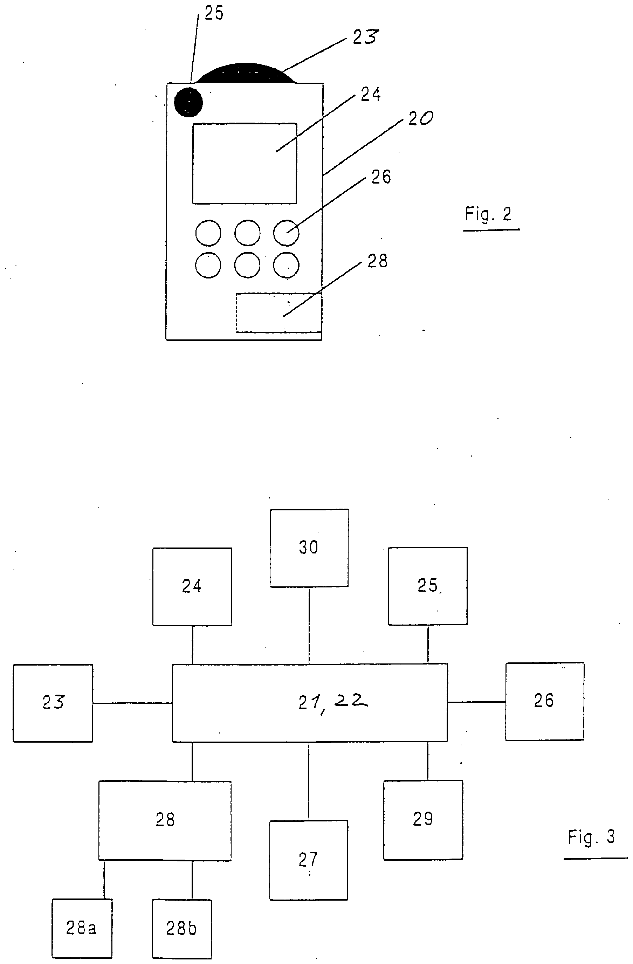

Figur 2 zeigt eine Vorderansicht des Kommunikations-Endgeräts 20. Sämtliche Komponenten des Kommunikations-Endgeräts 20 sind in einem leichten, in einer Hand haltbaren Gehäuse aufgenommen. Über die Schnittstelle 23 werden die Messignale P und F sowie Stellsignale C mit der Schnittstelle 13 ausgetauscht. Eine optische Anzeige 24 stellt für den Benutzer relevante Informationen dar. Dargestellte oder in einem Anzeigewahlmodus auf Abruf darstellbare Informationen sind zumindest Betriebsparameter des Verabreichungsgeräts 10. Die Ablesbarkeit wird insbesondere bei weiterlaufender Verabreichung durch die auch dabei bequem in der Hand haltbare Fernanzeige erheblich verbessert. Die Vielfalt und auch die Komplexität der angezeigten Information kann erhöht werden, ohne das Gewicht und die Abmesssungen des Verabreichungsgeräts vergrößern zu müssen.

Die Handhabbarkeit wird weiter noch dadurch verbessert, dass das Kommunikations-Endgerät 20 nicht nur als Fernanzeige, sondern auch als Fernbedienung ausgebildet ist. Hierzu weist das Endgerät 20 Eingabemittel 26 in Form von Tasten auf, die ein Einwirken auf den Prozessor 21 und auch auf die durch ihn gebildete Steuerung 22 ermöglichen. So kann durch Drücken auf eine der Tasten eine spontane oder eine in Abhängigkeit von der Eingabe zeitverzögert vorprogrammierte Bolusabgabe ausgelöst werden. Die optische Anzeige 24 und die Tasten 26 können auch zusammenwirken, indem auf der Anzeige 24 beispielsweise Tasteneinstellungen oder durch die Tasten 26 auswählbare Informationen angezeigt werden. Ferner weist das Kommunikations-Endgerät 20 ein akustisches Alarmmittel 25 auf, das gefährliche Fehlfunktionen des Kommunikations-Endgeräts 20 und auch des Verabreichungsgeräts 10 akustisch zur Kenntnis bringt.

Bezüglich der Bedienfunktion kann mittels des Endgeräts 20 das Verabreichungsgerät 10 ein-und ausgeschaltet und eine Bolusabgabe ausgelöst oder zumindest erhöht und erniedrigt werden. Ferner können die Basalrate und die temporäre Basalrate eingestellt und vorzugsweise auch verstellt werden. Auch die Befüllung des Katheters nach einem Ampullenwechsel kann derart kontrolliert erfolgen.

In Figur 3 sind die wesentlichen Komponenten des Kommunikations-Endgeräts 20 in Form eines Blockdiagramms miteinander verknüpft. Als zentraler Baustein steuert der Mikroprozessor 21 sowohl die optische Anzeige 24 als auch die akustische Anzeige 25 in Abhängigkeit von Eingangssignalen, die er von der Schnittstelle 23 oder dem Eingabemittel 26 erhält. Angedeutet ist auch eine Energiequelle 27.

Das Endgerät 20 ist mit einer Überwachungseinrichtung 29 zur Überwachung der Lage des Schrittmotors 9 ausgestattet. Hierzu erhält es die Istposition P des Lagesensors 14 über die Schnittstelle 23 zusammen mit dem Sollwert von der Prozessorsteuerung 22. Wird eine vorgegebene Höchstdifferenz dem Betrage nach überschritten, so wird durch das akustische Alarmmittel 25 ein Alarmsignal abgegeben. Noch tolerierbare Abweichungen werden angezeigt und können vom Benutzer über die Fernbedienung bei sich bietendem Anlass berücksichtigt und ausgeglichen werden, beispielsweise mit einer Sonderbolusabgabe. Eine Regelung erfolgt nicht. Der Soll/Ist-Vergleich, der lediglich der Sicherheit dient, wird bei einem Ausfall der Prozessorsteuerung 22 auch von der Notsteuerung 11 durchgeführt, wobei eine nicht tolerierbare Abweichung dann durch das Alarmmittel 17 zur Kenntnis gebracht werden kann.

Der Mikroprozessor 21 hat auf einen eigenen Speicher 30 des Kommunikations-Endgeräts 20 Zugriff, in dem insbesondere ein personenindividueller Setup und die historischen Daten der Verabreichung gespeichert sind bzw. ständig akkumulierend weitergespeichert werden. Auch Blutzuckermesswerte können über der Zeit gespeichert werden, entweder in einem eigenen Bereich des Speichers 30 oder in einem weiteren endgeräteeignen Speicher. So kann der Benutzer die Historie der Verabreichung mit den im Zeitverlauf zugeordneten, gespeicherten Blutzuckermesswerten vergleichen. Hierdurch kann der Benutzer für ihn wertvolle Rückschlüsse und gegebenenfalls Folgerungen für die zukünftige Verabreichung und individuelle Einstellung seines Verabreichungsgeräts ziehen, Das Kömmunikations-Endgerät 20 ist das elektronische Tagebuch des Benutzers.

Eine Blutzuckermesseinrichtung ist im Gehäuse des Kommunikations-Endgeräts 20 ebenfalls integriert. Die Blutzuckermesseinrichtung weist einen Sensor 28a und eine Messwertaufnahme 28b auf. Der Sensor 28a misst den Blutzuckergehalt einer Blutprobe und/oder Zellflüssigkeitsprobe. Die Messwertaufnahme 28b übernimmt ein vom Sensor 28a ausgegebenes Messignal, dessen Größe vom Blutzuckergehalt der Probe abhängt, und führt es dem Mikroprozessor 21, d.h. einer vom Mikroprozessor gebildeten Auswerteeinrichtung 28, zu. Der von dem Prozessor daraus gewonnene Messwert wird im Speicher 30 gespeichert, um so auch für eine spätere Darstellung an der Anzeige 24 verfügbar zu sein. Bei dem Sensor 28a handelt es sich um einen üblichen Sensor, insbesondere in Form eines Plättchens, mit einem Probenbereich zum Aufbringen der Probe und einem Kontaktbereich zum Einführen und zur Kontaktherstellung in und mit der Messwertaufnahme 28b. Die Blutzuckermesseinrichtung besteht aus dem Sensor 28a, der Messwertaufnahme 28b als Aufnahme- und Kontakteinrichtung für den Sensor 28a, einem Anschluss zum Verbinden mit dem Prozessor 21 und dem Prozessor 21 selbst, der anwendungsspezifisch programmiert die Auswertefunktion erfüllt und dem im Ausführungsbeispiel alle weiteren im Zusammenhang mit der Auswertung und Darstellung der Messignale des Sensors 28a stehenden Aufgaben zufallen.
Die Blutzuckermesseinrichtung kann komplett, wie vorstehend beschrieben, oder zu einem Teil als eigenständiges Modul ausgeführt sein, das in einen vorbereiteten Steckplatz des Kommunikations-Endgeräts 20 eingesteckt und dabei mit dem Prozessor 21 verbunden wird. Die Auswerteeinrichtung 28 kann durch einen Wandler gebildet werden, falls sie auf dem lösbaren Modul integriert ist, der das vom Sensor 28a übernommene Messsignal in ein vom Prozessor 21 lesbares Signal umwandelt. Die Auswerteeinrichtung 28 kann ein vom Sensor 28a übernommenes Messignal in einer bevorzugten weiteren Ausführungsform selbst in den Speicher 30 abspeichern, so dass der Prozessor 21 von der Aufgabe der Abspeicherung entlastet wird. Es ist auch möglich, die Ermittlung des Blutzuckergehalts in solch ein Messwertaufnahme- und Auswertemodul zu verlagern. Auch ein Speicher kann Bestandteil des Moduls sein, um die Sensormessignale darin Zwischenspeichern zu können. Eine Messeinrichtung in der Grundform als reine Messwertaufnahme 28b oder in einer der vorgenannten Ausbaustufen kann mit Vorteil bei jeder Art einer Vorrichtung, insbesondere einer gattungsgemäßen Vorrichtung ohne Betriebsparameteranzeige, verwendet werden, beispielsweise zusammen mit einer reinen Fernbedienung oder einer Fernanzeige mit anderen angezeigten Informationen. In der Ausführungsform als lösbares Modul kann sie in der vorgenannten Grundform oder in einer der vorgenannten Ausbaustufen mit Vorteil auch ein eigenständiges, von einer bestimmten Vorrichtung zur Verabreichung eines Produktfluids unabhängiges Produkt sein. Sie kann in dieser Form besonders bequem jede von einem Benutzer überwachte Therapie unterstützen. Sie kann eine eigene Anzeige aufweisen, um auch ohne Computer wie herkömmliche Auswerteeinrichtungen verwendbar zu sein.

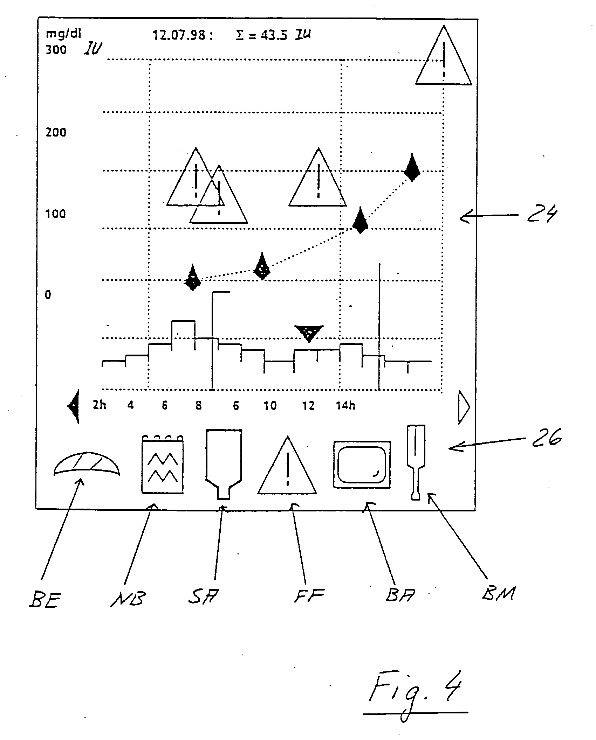

Figur 4 zeigt eine Darstellung, wie sie nach Anwählen eines geladenen Diabetesprogramms auf einem Touchscreen eines Palm Top Computers erscheint. Dessen Display ist Grafikanzeige und Eingabemittel zugleich.

Permanent dargestellt wird die im Verlaufe der letzten Stunden akkumuliert pro Stunde als Basalrate verabreichte Insulinmenge in Form eines Säulendiagramms in Insulineinheiten pro Stunde. Auch Sonderbolusabgaben, veranlasst durch den Benutzer, werden permanent dargestellt. Dabei steht ein einfacher senkrechter Strich für einen normalen Bolus und ein oben abgewinkelter Strich für einen verlängerten Bolus. Die mit dem Bolus verabreichte Menge wird durch die senkrechte Strichlänge angezeigt. Auf der Zeitachse ist der Zeitraum der letzten Stunden, beispielsweise der letzten vierundzwanzig Stunden, mit Uhrzeitangabe aufgetragen. Die verabreichten Insulinmengen werden vom Prozessor 21 aus den Istpositionen P ermittelt und im Speicher 30 abgelegt. Sie stehen so jederzeit für Darstellungs- und sonstige Auswertungszwecke zur Verfügung. Solange die Überwachungseinrichtung 29 keine Fehlfunktion feststellt, können statt der Istpositionen P auch einfach die Sollpositionen von der Steuerung 21 übernommen und zur Ermittlung der geförderten Insulinmengen verwendet werden. Schließlich wird die in den letzten vierundzwanzig Stunden vor dem Ablesen verabreichte Insulingesamtmenge in Form ihres numerischen Werts hinter einem Summenzeichen permanent angezeigt.

Unterhalb des Anzeigebereichs weist das Display einen Eingabebereich mit Eingabefeldern 26 auf. Jedes der Eingabefelder 26 ist mit einem die jeweilige Funktion darstellenden grafischen Symbol belegt. Von links nach rechts haben die Eingabefelder 26 die Bedeutung: Broteinheiten BE, Notizbuch NB, Ampullenstatus SA, Fehlfunktionen FF, Blutzuckeranzeige BA und Blutzuckermessung BM. Durch Druck auf eines der Felder wird dessen Funktion aktiviert, durch nochmaliges Drücken desaktiviert. Es kann jede beliebige Kombination von Feldern gleichzeitig aktiv sein. Figur 4 zeigt das Display in einem Zustand, in dem nur die Felder FF und BA akiviert sind.

Durch Druck auf das Feld BE wird die manuelle Eingabe der aufgenommenen Broteinheiten mit Zeit- und Mengenangabe möglich. Durch Druck auf das Feld NB kann der Benutzer ihm wichtig erscheinende persönliche Notizen eingeben, die mit dem vom Benutzer eingegebenen Zeitpunkt abgespeichert werden oder, falls der Benutzer die Zeit nicht definiert, automatisch mit dem Zeitpunkt der Aktivierung des Felds NB. Gleichzeitig werden auf dem Display dem betätigten Eingabefeld entsprechend die für den dargestellten Zeitraum eingegebenen Broteinheiten oder Notizen zeitecht dargestellt. Bei Drücken des Feldes SA wird der aktuelle Füllzustand der Ampulle in Prozent der Füllmenge einer neuen Ampulle und/oder als noch verbleibende Restmenge Insulin oder als geschätzte Restförderdauer angezeigt. Ebenso kann zusätzlich der Zeitpunkt des letzten Ampullenwechsels angezeigt werden. Bei Betätigung des Felds FF wird eine Abweichung vom Sollbetrieb, beispielsweise eine Okklusion, eine Kommunikationsstörung oder ein elektrischer Ausfall, zeitecht innerhalb des Warnsymbols geeignet dargestellt. Drücken des Feldes BA bewirkt die Darstellung des aus Messungen gewonnenen Blutzuckergehalts in beispielsweise Milligramm Zucker pro Deziliter Blut zum Zeitpunkt der jeweiligen Messung. Diesen Zeitpunkt bestimmt der Benutzer durch Drücken des Felds BM. Denkbar wäre jedoch auch die automatische Bestimmung dieses Zeitpunkts.

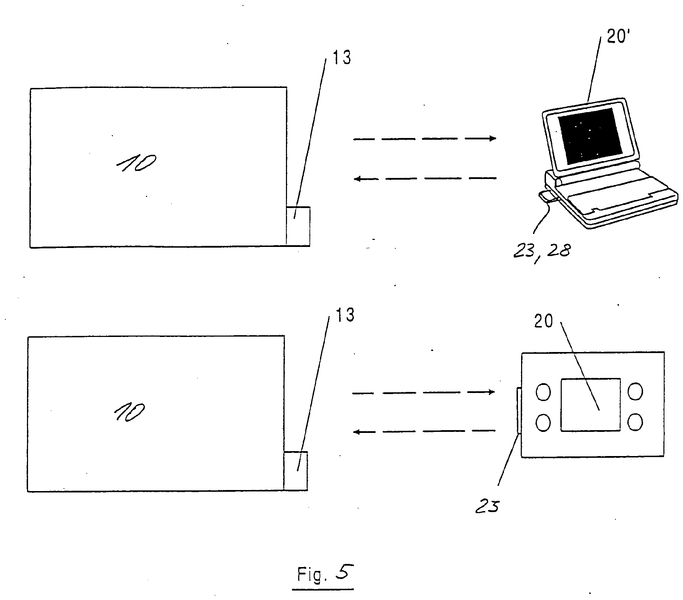

In Figur 5 ist neben der Möglichkeit der drahtlosen Kommunikation mit dem Endgerät 20 auch die Möglichkeit der Kommunikation über die gleiche Schnittstelle 13 mit einem Computer 20' dargestellt, in Figur 5 ein Notebook. Die Verwendung eines mit einem vergleichsweise großen Bildschirm ausgerüsteten Standard Computers 20' erhöht die Vielfalt und Komplexität der Verwendbarkeit der Fernanzeige und Fernbedienung erheblich. Mittels des PC's 20' kann das Verabreichungsgerät bis auf die personenindividuellen Einstellungen vollständig herstellerseitig konfiguriert werden, vorzugsweise durch Programmierung. Auch die individuelle Konfigurierbarkeit beim Benutzer vor Ort und insbesondere die Auswertemöglichkeiten des Benutzers, beispielsweise der Vergleich der Verabreichungshistorie mit den Blutzuckerwerten, kann besonders komfortabel und umfangreich gestaltet werden. Auch beim Hersteller kann die drahtlose Kommunikation zur wirtschaftlichen Konfigurierung und/oder Qualitätskontrolle eingesetzt werden. So muss insbesondere zur Qualitätskontrolle nicht jeweils eine Drahtverbindung zwischen dem zu kontrollierenden Verabreichungsgerät und dem zu Kontrollzwecken eingesetzten Computer hergestellt werden. Die Qualitätskontrolle kann mittels der drahtlosen Kommunikation sogar im Vorbeifördern der Verabreichungsgeräte durchgeführt werden.

## Patentansprüche

1. System zum Fernbedienen und Überwachen einer Verabreichungsvorrichtung zur Selbstverabreichung eines Produktfluids mit:
einer Vorrichtung zur Selbstverabreichung des Produktfluids mit einer Vorrichtung zur Ermittlung wenigstens eines Betriebsparameters der Verabreichungsvorrichtung und einer Schnittstelle (13) für eine drahtlose Kommunikation mit einer Fernbedienung; und
einer Fernbedienung mit einem Mikroprozessor (21), einem Speicher (30) und einer Schnittstelle (23) zum Empfangen des Betriebsparameters des Verabreichungsgerätes und zum Übertragen eines Signals an die Verabreichungsvorrichtung, wobei die Fernbedienung eine Anzeige zur Überwachung des Verabreichungsgerätes durch den Benutzer aufweist,
wobei der Mikroprozessor (21) auf den Speicher (30) Zugriff hat, in weichem ein individualisierter Setup und historische Daten der Verabreichung gespeichert sind bzw. ständig akkumulierend weitergespeichert werden und Blutzuckermesswerte in einem eigenen Bereich des Speichers (30) oder in einem weiteren Speicher der Fernbedienung gespeichert werden, so dass ein Benutzer eine Historie der Verabreichung mit den im Zeitverlauf zugeordneten gespeicherten Blutzuckermesswerten gemeinsam darstellen und vergleichen kann, und wobei die Fernbedienung derart eingerichtet ist, dass die Anzeige der Fernbedienung die Fluidmenge über der Zeit als Förderrate aufgetragen darstellen kann.

2. System nach Anspruch 1, wobei die Fernbedienung eine Funk-Fernbedienung, eine Ultraschall-Fernbedienung oder eine Infrarot-Fernbedienung ist.

3. System nach einem der vorhergehenden Ansprüche, wobei die Vorrichtung zur Ermittlung wenigstens eines Betriebsparameters die Position des Fördermittels, die von dem Fördermittel ausgeübte Reaktionskraft oder die verabreichte Fluidmenge als Betriebsparameter ermittelt.

4. System nach einem der vorhergehenden Ansprüche, wobei die Anzeige der Fernbedienung eine optische und/oder akustische Anzeige zur Überwachung des Verabreichungsgerätes durch den Benutzer ist.

5. System nach einem der vorhergehenden Ansprüche, wobei das Verabreichungsgerät eine Infusionspumpe (10) ist.

6. System nach dem vorhergehenden Anspruch, wobei die Infusionspumpe ein Reservoir für ein Produktfluid, insbesondere für Insulin, umfasst.

7. System nach einem der beiden vorhergehenden Ansprüche, wobei eine Fluidaustrittsstelle eine Einstechnadel ist.

8. System nach einem der drei vorhergehenden Ansprüche, wobei die Infusionspumpe (10) ein Gehäuse (1) aufweist, das mit Haltemitteln versehen ist, so dass es von einem Benutzer an der Kleidung oder unmittelbar am Körper zum ständigen Tragen befestigt werden kann.

9. System nach einem der vorhergehenden Ansprüche mit einem Sensor (28a), welcher an einer Person zur Ermittlung des Gesundheitszustandes angebracht werden kann und ein Messsignal sendet.

10. System nach dem vorhergehenden Anspruch, wobei der Sensor (28a) den Blutzuckergehalt einer Blutprobe messen kann.

11. System nach einem der zwei vorhergehenden Ansprüche, wobei der Sensor ein Okklusionssensor oder ein Leck-Detektions-Sensor ist.

12. System nach einem der drei vorhergehenden Ansprüche, wobei der Sensor einen Messwert drahtlos als Messsignal senden kann.

13. System nach dem vorhergehenden Anspruch mit einer Auswerteeinrichtung (28), welche das gesendete Messsignal empfängt und in ein von einem Computer verarbeitbares Signal umwandelt.

14. System nach dem vorhergehenden Anspruch, wobei die Auswerteeinrichtung (28) einen aufgenommenen Messwert auswertet, in dem daraus ein entsprechender Körperwert ermittelt wird.

15. System nach einem der vorhergehenden Ansprüche mit einem Speicher zur Speicherung des Messsignals.

16. System nach einem der vorhergehenden Ansprüche, wobei die Auswerteeinrichtung (28) einen Prozessor (21) umfasst zur Ermittlung einer den Gesundheitszustand der Person charakterisierenden Größe aus dem Messsignal.

17. System nach einem der vorhergehenden Ansprüche, wobei das Messsignal zur Ermittlung der Glukosekonzentrationen in einer Körperflüssigkeit oder des Hormonspiegels dient oder eine Körpertemperatur ist oder eine Kombination dieser Messsignale aufgenommen wird.

18. System nach einem der vorhergehenden Ansprüche mit einem Alarmmittel (17, 25), um die Lage eines Schrittmotors (9) oder einen Ausfall einer Prozesssteuerung (22) durch ein Alarmsignal anzeigen.

19. System nach einem der vorhergehenden Ansprüche, wobei das Kommunikations-Endgerät als Eingabemittel Tasten (26), einen Touchscreen oder eine Spracheingabe mit einem integrierten Mikrofon aufweist.

20. System nach einem der vorhergehenden Ansprüche mit einer Notsteuerung im Verabreichungsgerät (10), die bei einem Ausfall der Kommunikation ein festes Verabreichungsprogramm automatisch und autark bis zu einem Abschalten weiter fahren kann.

## Claims

1. System for remote control and monitoring of a delivery device for self-administration of a fluid product with:
a device for self-administration of the fluid product with a device for determining at least one operating parameter of the delivery device and an interface (13) for wireless communication with a remote control; and
a remote control with a microprocessor (21) a memory (30) and an interface (23) for receiving the operating parameter of the delivery device and for transmitting a signal to the delivery device, wherein the remote control includes a display for monitoring the delivery device by the user,
wherein the microprocessor (21) has access to the memory (30), in which an individualised setup and historical data regarding the administration is stored and constantly accumulated, and blood glucose readings are stored in a dedicated area of the memory (30) or in another memory on the remote control, so that a user can display a history of the administration together with the saved blood glucose readings assigned over time and compare them, and wherein the remote control is configured in such manner that the remote control display is able to represent the quantity of fluid as a delivery rate plotted over time.

2. System according to Claim 1, wherein the remote control is a wireless remote control, an ultrasonic remote control or an infrared remote control.

3. System according to one of the preceding claims, wherein the device for determining at least one operating parameter determines the position of the delivery means, the reaction force exerted by the delivery means or the quantity of fluid administered as an operating parameter.

4. System according to any one of the preceding claims, wherein the remote control display is an optical and/or acoustic display for monitoring the administration device by the user.

5. System according to any one of the preceding claims, wherein the delivery device is an infusion pump (10).

6. System according to the preceding claim, wherein the infusion pump comprises a reservoir for a fluid product, in particular for insulin.

7. System according to either of the two preceding claims, wherein a fluid exit site is a puncture needle.

8. System according to any of the three preceding claims, wherein the infusion pump (10) has a housing (1) which is provided with retaining means so that it can be attached to the clothing or directly to the body by a user for continuous wear.

9. System according to any one of the preceding claims, with a sensor (28a) which can be attached to a person to determine the person's health status, and which sends a measurement signal.

10. System according to the preceding claim, wherein the sensor (28a) is able to measure the blood glucose content of a blood sample.

11. System according either of the two preceding claims, wherein the sensor is an occlusion sensor or a leak detection sensor.

12. System according to any of the three preceding claims, wherein the sensor can send a measurement value wirelessly as a measurement signal.

13. System according to the preceding claim, with an evaluation device (28) which receives the transmitted measurement signal and converts it into a signal that can be processed by a computer.

14. System according to the preceding claim, wherein the evaluation device (28) evaluates a recorded measurement value by determining a corresponding body value therefrom.

15. System according to any one of the preceding claims, with a memory for storing the measurement signal.

16. System according to any one of the preceding claims, wherein the evaluation device (28) comprises a processor (21) for determining a variable which characterizes the state of health of the person from the measurement signal.

17. System according to any one of the preceding claims, wherein the measurement signal is used to determine the concentrations of glucose in a body fluid or of the hormone level, or is a body temperature, or a combination of these measurement signals is recorded.

18. System according to any one of the preceding claims, with an alarm means (17, 25) for reporting the location of a step motor (9) or a failure of a process controller (22) via an alarm signal.

19. System according to any one of the preceding claims, wherein the communication terminal device is equipped with keys (26), a touchscreen or voice input with a built-in microphone as input means.

20. System according to any one of the preceding claims, with an emergency controller in the delivery device (10) which can continue a fixed administration regimen automatically and autarchically until it is switched off in the event of a communication failure.

## Revendications

1. Système de commande à distance et de surveillance d'un dispositif d'administration pour l'auto-administration d'un produit liquide avec :
un dispositif d'auto-administration du produit liquide avec un dispositif pour déterminer au moins un paramètre de fonctionnement du dispositif d'administration et une interface (13) pour une communication sans fil avec une commande à distance, et
une commande à distance avec un microprocesseur (21), une mémoire (30) et une interface (23) pour réceptionner le paramètre de fonctionnement de l'appareil d'administration et pour transférer un signal au dispositif d'administration, la commande à distance comportant un affichage pour la surveillance de l'appareil d'administration par l'utilisateur,
le microprocesseur (21) ayant accès à la mémoire (30) dans laquelle sont mémorisés ou ultérieurement mémorisés en permanence par accumulation un réglage individualisé et les données historiques de l'administration et des valeurs de mesure de glycémie sont mémorisées dans une zone propre de la mémoire (30) ou dans une autre mémoire de la commande à distance de telle sorte qu'un utilisateur peut représenter et comparer en commun un historique de l'administration avec les valeurs de mesure de glycémie mémorisées attribuées au cours du temps et la commande à distance étant agencée de telle sorte que l'affichage de la commande à distance peut représenter la quantité de liquide dans le temps rapportée sous la forme de débit.

2. Système selon la revendication 1, la commande à distance étant une télécommande radio, une télécommande à ultrasons ou une télécommande à infrarouge.

3. Système selon l'une quelconque des revendications précédentes, le dispositif pour déterminer au moins un paramètre de fonctionnement déterminant la position du moyen de transport, la force de réaction exercée par le moyen de transport ou la quantité de liquide administrée en tant que paramètre de fonctionnement.

4. Système selon l'une quelconque des revendications précédentes, l'affichage de la commande à distance étant un affichage optique et/ou acoustique pour surveiller l'appareil d'administration par l'utilisateur.

5. Système selon l'une quelconque des revendications précédentes, l'appareil d'administration étant une pompe à perfusion (10).

6. Système selon la revendication précédente, la pompe à perfusion comprenant un réservoir pour un produit liquide, en particulier pour de l'insuline.

7. Système selon l'une des deux revendications précédentes, un point de sortie du liquide étant une aiguille à injection.

8. Système selon l'une quelconque des revendications précédentes, la pompe à perfusion (10) comportant un boîtier (1), qui est doté de moyens de fixation de telle sorte qu'il peut être fixé par un utilisateur au vêtement ou directement sur le corps pour le porter en permanence.

9. Système selon l'une quelconque des revendications précédentes avec un capteur (28a), lequel peut être monté sur une personne pour déterminer l'état de santé et envoie un signal de mesure.

10. Système selon l'une quelconque des revendications précédentes, le capteur (28a) pouvant mesurer la teneur en glycémie d'un échantillon sanguin.

11. Système selon l'une quelconque des revendications précédentes, le capteur étant un capteur d'occlusion ou un capteur de détection de fuite.

12. Système selon l'une quelconque des revendications précédentes, le capteur pouvant envoyer une valeur de mesure sans fil sous la forme d'un signal de mesure.

13. Système selon la revendication précédente avec un dispositif d'exploitation (28), lequel reçoit le signal de mesure envoyé et le transforme en un signal pouvant être traité dans un ordinateur.

14. Système selon la revendication précédente, le dispositif d'exploitation (28) exploitant une valeur de mesure enregistrée, une valeur corporelle correspondante étant déterminée à partir de celle-ci.

15. Système selon l'une quelconque des revendications précédentes avec une mémoire pour mémoriser le signal de mesure.

16. Système selon l'une quelconque des revendications précédentes, le dispositif d'exploitation (28) comprenant un processeur (21) pour déterminer une grandeur à partir du signal de mesure, caractérisant l'état de santé de la personne.

17. Système selon l'une quelconque des revendications précédentes, le signal de mesure sert à déterminer les concentrations de glucose dans un liquide corporel ou du niveau hormonal ou est une température corporelle ou une combinaison de ces signaux de mesures est enregistrée.

18. Système selon l'une quelconque des revendications précédentes avec un moyen d'alarme (17,25) pour afficher la situation d'un moteur pas à pas (9) ou une défaillance d'une commande de processus (22) par un signal d'alarme.

19. Système selon l'une quelconque des revendications précédentes, le terminal de communication comportant des touches comme moyens d'introduction (26), un écran tactile ou une entrée vocale avec in microphone intégré.

20. Système selon l'une quelconque des revendications précédentes avec une commande de secours dans l'appareil d'administration (10), qui en cas d'une défaillance de la communication peut poursuivre automatiquement et indépendamment le programme d'administration jusqu'à un arrêt.
